Europäisches Patentamt

(19) European Patent Office

Office européen des brevets

(11) Publication number: **0 078 093**
**B1**

(12) **EUROPEAN PATENT SPECIFICATION**

(45) Date of publication of patent specification: **01.07.87**

(51) Int. Cl.⁴: **A 61 H 1/02**

(21) Application number: **82302019.3**

(22) Date of filing: **20.04.82**

(54) Device for imparting continuous passive motion to human joints.

(30) Priority: **23.10.81 CA 388659**

(43) Date of publication of application:
**04.05.83 Bulletin 83/18**

(45) Publication of the grant of the patent:
**01.07.87 Bulletin 87/27**

(84) Designated Contracting States:
**AT BE CH DE FR GB IT LI LU NL SE**

(56) References cited:
**DE-C- 191 678**
**FR-A-2 109 426**
**US-A-3 323 518**
**US-A-3 824 994**
**US-A-3 976 057**

(73) Proprietor: **TORONTO MEDICAL CORP.**
**27 Golden Gate Crt.**
**Scarborough, Ontario M1P 3A4 (CA)**

(72) Inventor: **Saringer, John H.**
**32 Gifford Street**
**Toronto Ontario M5A 3J1 (CA)**

(74) Representative: **Smith, Norman Ian et al**
**F.J. CLEVELAND & COMPANY 40-43 Chancery Lane**
**London WC2A 1JQ (GB)**

## Description

This invention relates to a prosthetic device used to treat a human joint by applying "continuous passive motion".

Dr. Robert B. Salter, Professor and Head of Orthopaedic Surgery at the University of Toronto, and Senior Orthopaedic Surgeon at the Hospital for Sick Children in Toronto, first developed the concept and coined the expression "continuous passive motion". Dr. Salter's work is described in the article "Joints Were Meant to Move — and Move Again" by Ohlendorf in "The Graduate", published by The Department of Information Services, University of Toronto, September/October 1980.

Briefly, according to this concept, a human joint, for example, a knee, elbow, or finger joint, is kept under slow continuous constrained motion as distinct from being held motionless or being moved intermittently. Keeping an injured or post operative joint mobile rather than immobilizing it in a cast is beneficial to the cartilage.

Attempts which have been made to provide machines that exercise joints are designed for intermittent operation and do not supply continuous passive motion. Moreover, they are usually too heavy and bulky to be readily portable and thus to be mounted on the body.

A known apparatus for mobilizing a human joint is summarised in the precharacterising portion of Claim 1 and is also known from US—A—3976057 and US—A—3323518. US—A—3976057 describes a flexing apparatus for a knee in which straps on either side of the knee are connected firstly by a pivoted joint and secondly by a piston and cylinder assembly extending between the two straps to cause mobilization of the joint about the pivot point. By use of mechanical members, the extension limit of the apparatus can be limited. By use of an air supply, a powered embodiment of the apparatus can be produced. US—A—3323518 describes a structure wherein a flexing arrangement is fixed between two straps provided either side of an elbow joint. The flexing apparatus is driven by a flexible shaft extending from a motor. The flexing arrangement comprises a drive screw having both left and right hand threads about which is provided a drive collar the movement of which is fixed to urge flexing of the elbow joint. The motor drives the double threaded screw to move the collar forwards and backwards. Reversal of the movement of the collar is achieved by joining the right and left hand threads at opposite ends of the drive screw so that a continuous path is traversed to provide a continuous, slow periodic movement.

The present invention is summarised by the characterising portion of Claim 1 and allows the traveller means to reach the end of its stroke thereby providing the rythmic cycle, but advantageously where there is some unplanned obstruction, for example, sheets jamming in the mechanism or where the human patient offers undue resistance, the means responsive to a predetermined load acts as a safety function as well as the prime actuating function.

Examples of the present invention will now be described with reference to the accompanying drawings, in which:—

*Figure 1* is a schematic showing apparatus for treating a human elbow joint of a patient.

*Figure 2* is a greatly enlarged fragmentary view showing the wrist connection in the apparatus of Figure 1.

*Figure 3* is a schematic perspective exploded diagram, showing the arrangement of a part of the apparatus of Figure 1 and 2.

*Figure 4* is a side elevation showing apparatus for treating a human knee joint in which the apparatus forms part of a crutch;

*Figure 5* is a side elevation of a variation of the apparatus of Figure 4;

*Figure 6* is a fragmentary perspective showing an apparatus for treating the said knee joint and which is connected to a bed on which the patient is placed;

*Figure 7* is a fragmentary view showing a unit for treating a human finger or thumb joint;

*Figure 8* is a view in oblique perspective of a housing shown in Figure 7, illustrating the drive mechanism.

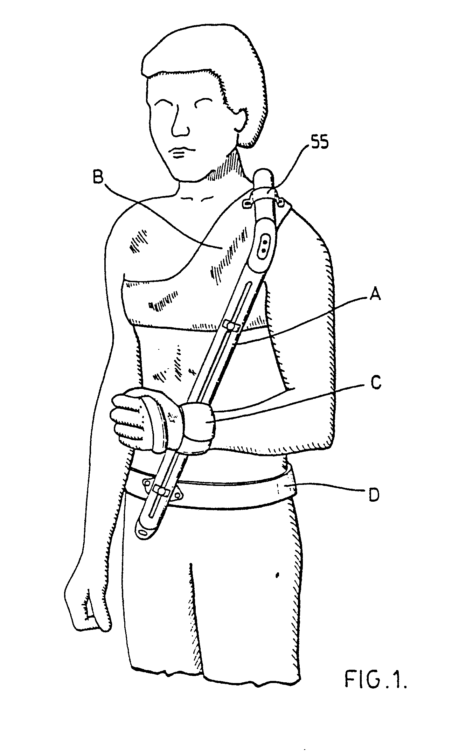

Referring more particularly to Figure 1, apparatus is shown made up of a support structure of goose neck form which, in this case, is an elongated housing A connected to a human shoulder for treating the human elbow joint by a harness B and to the waist by a belt D. A traveller means (not shown) movable to the housing, is connected to the wrist by a cuff C. The traveller means is supported by the housing for linear reciprocating movement relative to it. Motor means drives the traveller means back and forth through a predetermined stroke in a slow rhythmic cycle. This imparts a corresponding reciprocating motion to the arm thus imparting continuous passive motion to the elbow joint.

The speed of movement of the traveller means would, in the embodiment shown, range from one-half foot (152.4 mm) to four feet (1219 mm) per minute, or from one-third to two cycles per minute.

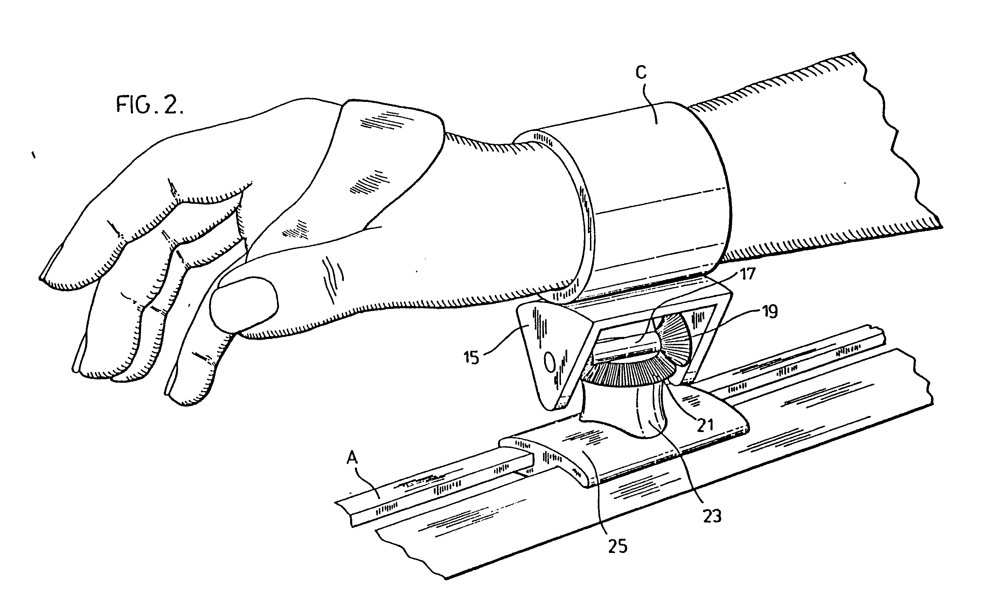

In order to supply supination and pronation to the human wrist, the cuff C is connected (see Figure 2) to a special mechanism that is mounted for rotation on a U-shape bracket 15 a shaft 17, carrying a bevel gear 19, that meshes with a crown gear 21, carried on a hub 23, mounted on a plate 25 froming a part of the traveller means.

The plate 25 moves back and forth, along the housing A, and flexing of the human elbow causes a small rotation at movement of the human arm at the wrist, by virtue of its connection to the bevel gear 19 as it is caused to ride around the crown gear 21. At the same time, the wrist is held to the plate 25 so that it makes longitudinal movements with the latter along the housing A.

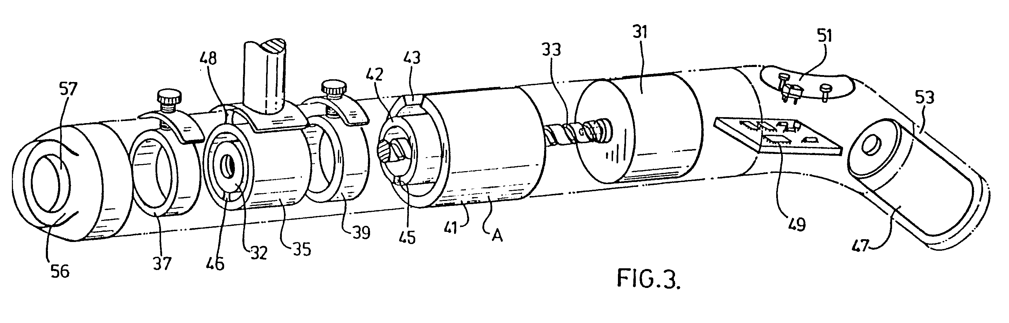

Figure 3 shows in detail the support housing, actuating means, motor means and associated parts. A motor 31 drives an elongated screw 33 whose opposite end is rotatably held in a recirculating ball-type nut 32. A cylindrical slide 35 is connected to the nut 32 and moves linearly under the drive of the screw 33 between the limiting stops 37 and 39, which are locked to the housing A by thumb screws.

The mechanism is encased in an outer tube 41 and an inner concentric tube 42. The tube 41 is provided with a longitudinal slot 43 and the concentric tube 42 with an elongated slot 45. The slot 43; accommodate the flange connection 48 between the slides 35 and the plate 25. The slot 45 accommodates the flange connection 46 between the ball nut 33 and the slide 35.

The motor 31 is powered by batteries 47 which operate through circuits on a circuit board 49 and is governed by controls 51. The tube 41 is provided with a gooseneck part 53 about which there extends a bracket 55 connecting it to the harness B. The other end of the tube 41 has a terminal 56 provided with an eye 57 so that it can be hung from a suitable hook when required.

The motor 31 is a reversible motor. It will reverse as soon as an excessive load is applied. This may be, for example, where the traveller means reaches the end of its stroke and is halted by the stop 37 or 39 as the case may be. Or, it may be where there is some unplanned obstruction, for example, sheets jamming in the mechanism or where the human patient offers undue resistance. A safety function is thus performed as well as prime actuating function.

Examples of suitable electric motors are geared dc. Brush type motors made by Faulhaber, Escap, and Maxon Precision, all of Switzerland.

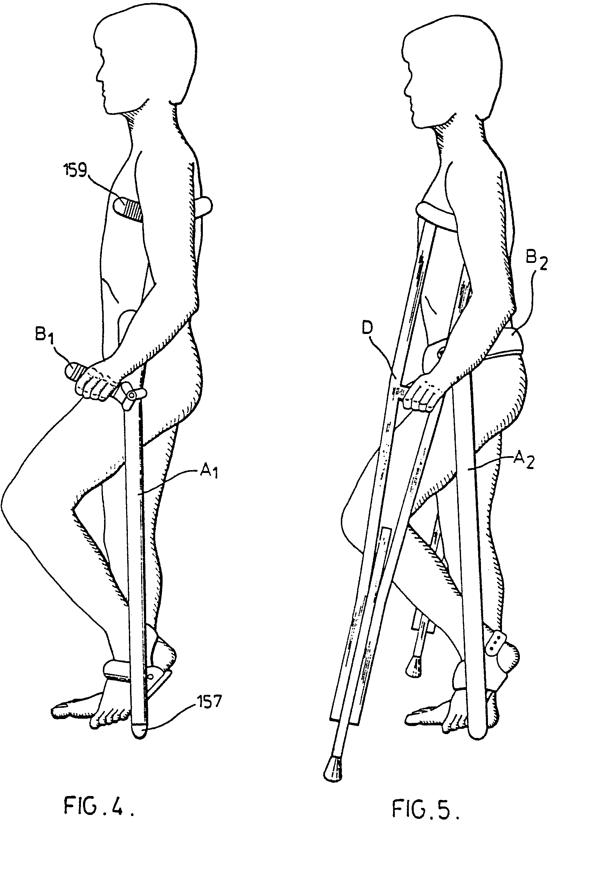

Figure 4 describes an apparatus for flexing the human knee joint. Similar numbers or letters have been applied to similar parts, as on Figures 1, 2 and 3, in Figure 4 and the following figures with the exception that they have been raised by 100 and the letters have been given a suitable subscript.

A support housing $A_1$ forms part of a crutch for supporting the patient and embodies operating mechanism similar to that shown in Figure 3, except that the support housing $A_1$ is a straight instead of having a gooseneck end. It has a rubber foot 156 taking the place of the terminal 56. A handle $B_1$ takes the place of the harness B and is provided with an armpit bar 159. The handle $B_1$ slides on the top end of the support housing $A_1$ to give adjustability. Taking the place of the plate 25 that moves back and forth is a simple foot plate $25_1$ with staps, similar to the supination and pronation mechanism for rotating the wrist of Figure 2.

Figure 5 shows an alternative arrangement in which a waistband $B_2$ is employed to connect the top of a support housing $A_2$ to the body. A separate crutch D supports the patient and the foot plate is now numbered $25_2$.

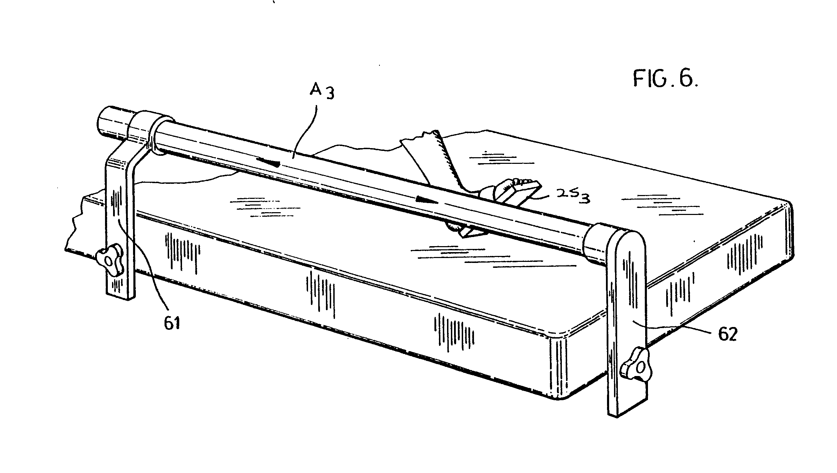

Figure 6 illustrates a further form of leg exercising device. In this case, the support housing $A_1$, $A_2$ of Figures 4 and 5 is connected to a bed and is as support housing $A_3$ retained by spaced-apart brackets 61 and 62. The leg of a patient lying on the bed is connected to a movable foot plate $25_3$ as in Figures 4 and 5.

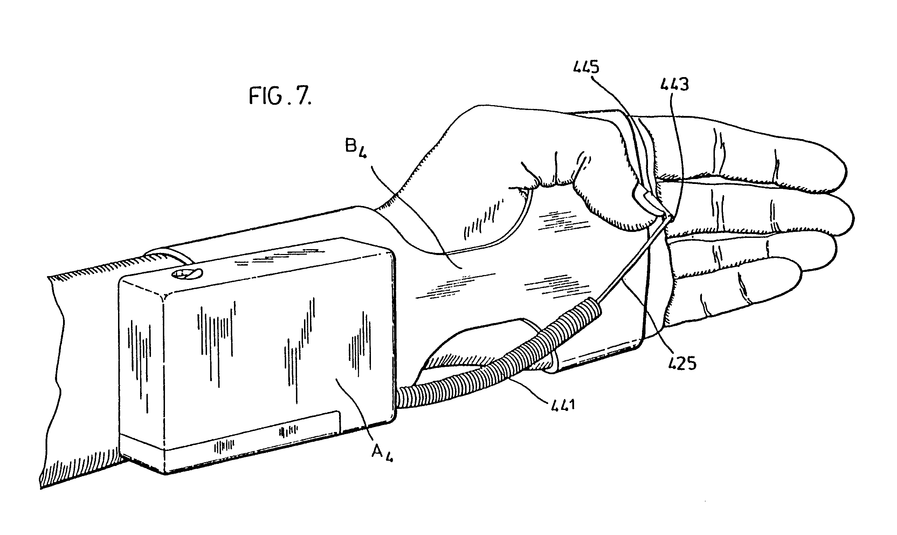

Figure 7 illustrates a device for flexing human finger and thumb joints. Here again a support housing $A_4$ is connected to a cuff $B_4$ mounted on a wrist and hand in place of the harness B of Figure 1. An actuator wire 425 moves back and forth from the housing $A_4$, through a flexible guide tube 441, to a plate 445 on the thumb via a hinge 443.

Clearly, the actuator member 445 can be connected to any one of the fingers or several fingers at a time.

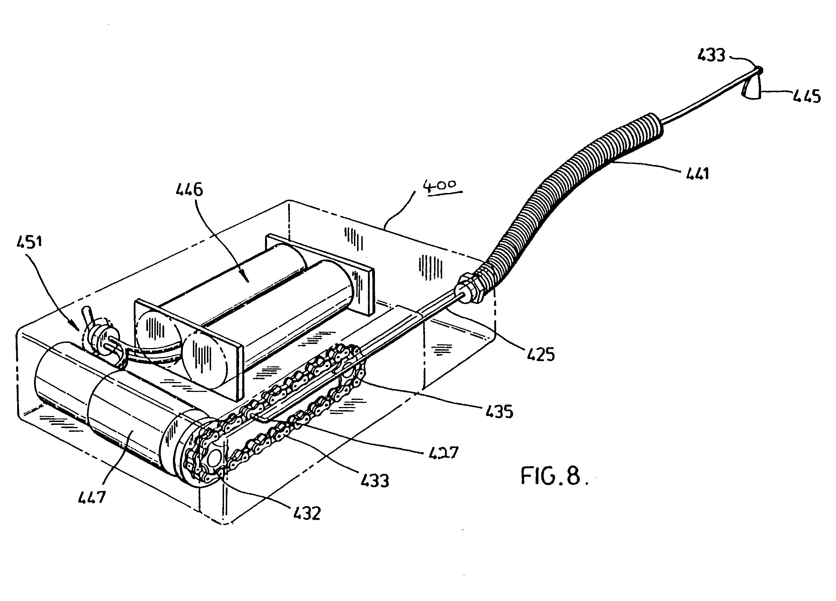

A mechanism for moving an actuator wire 425 is shown in Figure 8. The support structure is fashioned from a block of plastic material shown generally at *400* made to accommodate the various parts. A geared motor 447 drives a sprocket 432 about which there is connected a sprocket chain 433 which is also about a spaced-apart sprocket 435. The actuator wire 425 is connected at 427 to one ofthe links of the said sprocket chain 433. Batteries 446 are accommodated within the block 400 as is an operating switch 451. The geared motor moves the sprocket chain 435 continuously so that the actuator wire 425 moves in one direction along the top run of the chain and then down along the bottom run in the other direction so as to impart substantially continuous reciprocating movement to the wire 425 and consequently to the finger joints of the human hand of Figure 7.

**Claims**

1. Apparatus for mobilizing a human joint comprising:—
   flexing means;
   connecting means (c) for connecting the flexing means to operatively engage a limb of the body connected to the joint to be mobilized;
   motor means (31) for driving the flexing means to move back and forth through a reciprocating linear stroke in a rythmic cycle whereby the limb is moved to mobilize said joint; characterised by
   the flexing means comprising an elongated support (A) and traveller means (25) provided on the support for reciprocating linear movement relative thereto; and by the motor means (31) having means responsive to a predetermined load to reverse the direction of travel of the traveller means at any point in its path of travel.

2. Apparatus as claimed in Claim 1 wherein the elongated support (A) extends between a support end and an opposite end; the apparatus further including
   supporting means for supporting the supported end from a support while leaving said opposite end for retention remote from said supported end;
   said connecting means connecting the traveller means to an extremity of a limb wherein a plurality of joints of the limb form a linkage intervening the supporting means and the extremity;
   and said motor means (31) being fixedly carried by the elongated support, and the transmission means (33) being carried by the support between said motor means and said travelling means so that the apparatus is portable.

3. Apparatus as claimed in Claim 1 or 2 wherein said linear stroke has a cycle of between one third and two cycles per minute.

4. Apparatus as claimed in any preceding claim wherein said motor means is provided at one end of the elongated support and substantially aligned therewith;

transmission means is provided to connect the motor means to drive the traveller means movement;

and means for reversing the motor in response to a predetermined load.

5. Apparatus as claimed in Claim 4 wherein the elongated support (A) includes an outer elongate tubular housing (41);

an inner housing (42) is mounted concentrically to the outer housing (41);

each said housing (41, 42) is provided with a longitudinally extending slot (43, 45);

a drive screw (33) is concentrically mounted within the inner housing (42);

said motor means comprises a geared motor (31) connected to one end of the drive screw (33) and a recirculating ball nut (32) is connected to the other end of the drive screw (33);

said traveller means comprising an annular traveller member (35) mounted to travel between the inner and outer housing (41, 42) and held to said ball nut (32) by attaching means (46) extending through the slot (45) in the inner member (42);

a connecting plate (25) mounted on the traveller member (35) by further attaching means (48) extending through the slot (43) in the outer housing (41);

a pair of spaced-apart terminal stops (37, 39) fixed to the outer housing (41) to limit the travel of the traveller member (35);

the motor being (31) reversible whereby it drives the drive screw first (33) in one direction then in the other to cause reciprocating movement of the travelling member (25).

6. The apparatus as claimed in Claim 5 wherein the change in direction of the motor is caused by predetermined resistance of the screw (33) by rotation.

7. The apparatus as claimed in Claim 5 wherein the motor is reversed in response to a predetermined load applied to the transmission means.

8. Apparatus as claimed in any one of claims 2—7 wherein the supporting means is connectable to the upper body of a patient and the connecting means is connectable to the wrist and includes means for converting elbow flexion to wrist rotation to cause thereby supination and pronation of the wrist.

9. Apparatus as claimed in Claim 8 wherein the connecting means connectable to the wrist includes a bracket (15) rotatably mounting a pinion (21) and a traveller plate (25) on the support carrying a bevel gear (19) meshing with the pinion (21) whereby flexion of the arm at the elbow causes rotation of the wrist about its axis.

10. Apparatus as claimed in Claim 1, wherein the connecting means comprises a handcuff connecting a support structure (A₄) to a hand and wherein the flexing means comprises a wire (425) running through a guide,

said motor means comprising a geared motor (447) and means (432, 433, 435) driven by the motor (447) to impart reciprocating movement to the wire (425);

an extremity of the wire (425) being connected to at least one hand digit.

11. An apparatus, as defined in Claim 10, in which the motor drives an endless chain (433) and the wire (425) is connected to said chain (433) to move therewith to describe a reciprocating movement.

12. Apparatus as claimed in any one of claims 1 to 9 wherein the motor means (31) is substantially aligned with said linear movement and is fixedly carried by the support to drive said traveller means movement whereby the apparatus is portable.

**Patentansprüche**

1. Einrichtung zum Betätigen eines menschlichen Gelenkes, umfaßt:
— Beugebetätigungsmittel
— Verbindungsmittel (C) zum Verbinden der Beugebetätigungsmittel in einer bewegungsübertragenden Weise mit einem Glied des Körpers, welches an dem zu betätigenden Gelenk angreift;
— Antriebsmittel (31) für den Antrieb der Beugebetätigungsmittel zu einer Vor- und Rückbewegung längs eines linearen, hin- und hergehenden Bewegungshubes in rythmischem Zyklus, wobei das Körperglied zum Zwecke der Betätigung des Gelenkes bewegt wird, dadurch gekennzeichnet, daß die Beugebetätigungsmittel einen länglichen Träger (A) und eine am Träger relativ zu diesem linear hin- und herbewegbar angeordnete Schlittenanordnung (25) umfassen, und daß die Antriebsmittel (31) auf eine vorbestimmte Last ansprechende Steuermittel umfassen, durch welche die Bewegungsrichtung der Schlittenanordnung an jedem Punkt der Bewegungsbahn umgekehrt wird.

2. Einrichtung nach Anspruch 1, dadurch gekennzeichnet, daß der längliche Träger (A) sich zwischen einem gehaltenen Ende und einem diesem abgewandten Ende erstreckt, wobei die Einrichtung ferner Befestigungsmittel zum Befestigen des gehaltenen Endes eines Trägers umfaßt, während das abgewandte Ende von dem gehaltenen Ende entfernt bleibt;
— daß die Verbindungsmittel die Schlittenanordnung mit einem Ende des Körpergliedes verbinden, wobei eine Mehrzahl von Gelenken des Körpergliedes eine Kopplung zwischen den Befestigungsmitteln und dem Körpergliedende herstellen;
— und daß die Antriebsmittel (31) am länglichen Träger fest angeordnet sind, wobei die Übertragungsmittel (33) am länglichen Träger zwischen den Antriebsmitteln und der Schlittenanordnung so angeordnet sind, daß die ganze Einrichtung tragbar ist.

3. Einrichtung nach Anspruch 1 oder 2, dadurch gekennzeichnet, daß der lineare Bewegungshub eine Wiederholdauer zwischem einem Drittel Zyklus und zwei Zyklen pro Minute aufweist.

4. Einrichtung nach einem der vorstehenden Ansprüche, dadurch gekennzeichnet, daß die Antriebsmittel an einem Ende des länglichen Trägers und im wesentlichen in dessen Längsachse angeordnet sind; daß die Übertragungsmittel die Antriebsmittel mit der Schlittenanordnung zur Bewegungsübertragung verbinden, und daß Mittel Umsteuerung der Antriebsmittel in Abhängigkeit von einer vorbestimmten Last vorgesehen sind.

5. Einrichtung nach Anspruch 4, dadurch gekennzeichnet, daß der längliche Träger (A) ein äußeres längliches, rohrförmiges Gehäuse (41) aufweist,

— daß ein inneres, rohrförmiges Gehäuse (42) konzentrisch zum äußeren Gehäuse (41) angeordnet ist,

— daß jedes der Gehäuse (41, 42) mit einem sich in Längsrichtung erstreckenden Schlitz (43, 45) versehen ist,

— daß eine Antriebs-Gewindespindel (33) konzentrisch innerhalb des inneren Gehäuses (42) angeordnet ist,

— daß die Antriebsmittel einen Getriebemotor (31) umfasen, welcher mit einem Ende der Gewindespindel (33) verbunden ist, und eine Rücklauf-Kugelmutter (32), welche mit dem anderen Ende der Gewindespindel (33) verbunden ist,

— daß die Schlittenanordnung ein ringförmiges Schlittenteil (35) umfassen, welches zwischen dem inneren und dem äußeren Gehäuse (41, 42) verfahrbar angeordnet und mit der Kugelmutter (32) durch Befestigungsmittel (46) verbunden ist, welche durch den Schlitz (45) am inneren Gehäuse (42) hindurchgreifen,

— daß am Schlittenteil (35) eine Verbindungsplatte (25) über weitere Befestigungsmittel (48) befestigt ist, welche durch den Schlitz (43) am äußeren Gehäuse (419) hindurchgreifen,

— daß zwei im Abstand voneinander angeordnete Endanschläge (37, 39) am äußeren Gehäuse (41) befestigt sind, um die Bewegung des Schlittenteils (35) zu begrenzen,

— und daß der Motor (31) reversibel ist, sodaß er die Gewindespindel (33) zuerst in einer Richtung und sodann in der anderen Richtung antreibt und so eine Hin- und Herbewegung des Schlittenteils (25) erzeugt.

6. Einrichtung nach Anspruch 5, dadurch gekennzeichnet, daß der Wechsel der Bewegungsrichtung des Motors (31) durch einen vorbestimmten Drehwiderstand der Gewindespindel (33) hervorgerufen wird.

7. Einrichtung nach Anspruch 5, dadurch gekennzeichnet, daß der Motor (31) in Abhängigkeit von einer vorbestimmten, auf die Übertragungsmittel wirkenden Last umgesteuert wird.

8. Einrichtung nach einem der Ansprüche 2 bis 7, dadurch gekennzeichnet, daß die Befestigungsmittel am Oberkörper eines Patienten befestigbar sind, daß die Verbindungsmittel am Handgelenk befestigbar sind und Mittel aufweisen, welche eine Beugebewegung des Ellenbogens in eine Drehung des Handgelenkes umwandeln und dadurch eine Auswärtsdrehung und eine Einwärtsdrehung (Supination und Pronation) des Handgelenkes bewirken.

9. Einrichtung nach Anspruch 8, dadurch gekennzeichnet, daß die an dem Handgelenk befestigbaren Verbindungsmittel eine Konsole (15) umfassen, ein daran drehbar angeordnetes Planetenrad (19) und eine am Träger (A) angeordnete Schlittenplatte (25), welche ein Kegelrad (21) trägt, das mit dem Planetenrad (19) kämmt, wodurch eine Beugung des Armes im Ellenbogen eine Drehung des Handgelenkes um seine Achse bewirkt.

10. Einrichtung nach Anspruch 1, dadurch gekennzeichnet, daß die Verbindungsmittel eine Armmanschette umfassen, mittels welcher eine Trägerstruktur ($A_4$) an einer Hand befestigt wird, und daß die Beugebetätigungsmittel einen Drahtzug (425) umfassen, welcher durch eine Führungshülse verläuft,

— daß die Antriebsmittel einen Getriebmotor (447) umfassen und Mittel (432, 433, 435), welche durch den Motor (447) angetrieben werden und auf den Drahtzug (425) eine Hin- und Herbewegung übertragen,

— und daß ein Ende des Drahtzuges (425) mit wenigstens einem Finger verbunden ist.

11. Einrichtung nach Anspruch 10, dadurch gekennzeichnet, daß der Motor eine endlose Kette (433) antreibt und der Drahtzug (425) mit dieser Kette (433) verbunden ist, sich mit dieser bewegt und einer Hin- und Herbewegung ausführt.

12. Einrichtung nach einem der Ansprüche 1 bis 9, dadurch gekennzeichnet, daß der Motor (31) im wesentlichen auf der linearen Bewegungsachse angeordnet und mit dem Träger zum Zwecke des Bewegungsantriebs der Schlittenanordnung fest verbunden ist, sodaß die gesamte Einrichtung tragbar ist.

**Revendications**

1. Appareil pour assurer la mobilisation d'une articulation humaine, comprenant: un dispositif de fléchissement; un dispositif de connexion (C) destiné à relier le dispositif de fléchissement pour qu'il coopère de façon active avec un membre du corps lié à l'articulation à mobiliser; un dispositif moteur (31) pour amener le dispositif de fléchissement à se déplacer en va-et-vient suivant une course linéaire alternative et suivant un cycle rythmique, de manière que le membre soit déplacé pour mobiliser l'articulation susdite, caractérisé en ce que le dispositif de fléchissement comprend un long support (A) et un élément mobile (25) monté sur le support en vue d'un mouvement linéaire de va-et-vient par rapport à celui-ci, et en ce que le dispositif moteur (31) comporte des moyens sensibles à une charge prédéterminée pour inverser le sens de déplacement de l'élément mobile en un point quelconque de son parcours de déplacement.

2. Appareil suivant la revendication 1, caractérisé en ce que le long support (A) s'étend entre une extrémité soutenue et une extrémité opposée, cet appareil comprenant en outre un dispositif de support destiné à soutenir l'extrémité supportée par un support, tout en laissant l'extrémité opposée susdite destinée à la rétention, à l'écart de l'extrémité supportée susdite; le dispositif de connexion susdit reliant l'élément mobile à une extrémité d'un membre où plusieurs articulations de ce membre forment une liaison intervenant entre le dispositif de support et l'extrémité; et le dispositif moteur susdit (31) est supporté de manière fixe par le long support, le dispositif de transmission (33) étant soutenu par le support entre le dispositif moteur et l'élément mobile de manière que l'appareil soit transportable.

3. Appareil suivant la revendication 1 ou 2, caractérisé en ce que la course linéaire susdite a un cycle compris entre un tiers et deux cycles par minute.

4. Appareil suivant l'une quelconque des revendications précédentes, caractérisé en ce que le dispositif moteur susdit est prévu à une extrémité du long support et est sensiblement en alignement avec lui, le dispositif de transmission est prévu pour connecter le dispositif moteur en veu de la commande du déplacement de l'élément mobile, cet appareil comportant des moyens pour inverser le moteur en réponse à une charge prédéterminée.

5. Appareil suivant la revendication 4, caractérisé en ce que le long support (A) comporte un logement tubulaire allongé extérieur (41), un logement tubulaire interne (42) monté concentriquement au logement externe (41), chaque logement susdit (41, 42) étant pourvu d'une fente longitudinale (43, 45), une vis de commande (33) est montée concentriquement à l'intérieur du logement interne (42), le dispositif moteur susdit comprend un moteur à engrenage (31) relié à une extrémité de la vis de commande (33) et un écrou à billes circulantes (32) est relié à l'autre extrémité de la vis de commande (33), l'élément mobile susdit comprenant un élément mobile annulaire (35) monté de manière à se déplacer entre le logement interne et le logement externe (41, 42) et maintenu sur l'écrou à billes (32) par un moyen d'attache (46) s'étendant à travers la fente (45) de l'élément de logement interne (42), une plaque de connexion (25) est montée sur l'élément mobile (35) par un autre moyen d'attache (48) s'étendant à travers la fente (43) du logement externe (41), une paire d'arrêts espacés (37, 39) étant fixés au logement externe (41) pour limiter le parcours de l'élément mobile (35), le moteur (31) étant réversible de sorte qu'il entraîné la vis de commande (33) d'abord dans un sens, puis dans l'autre sens pour assurer un mouvement de va-et-vient de l'élément mobile (25).

6. Appareil suivant la revendication 5, caractérisé en ce que le changement de sens du moteur est provoqué par une résistance prédéterminée de la vis (33) à la rotation.

7. Appareil suivant la revendication 5, caractérisé en ce que le moteur est inversé en réponse à une charge prédéterminée appliquée au dispositif de transmission.

8. Appareil suivant l'une quelconque des revendications 2 à 7, caractérisé en ce que le dispositif de support peut être relié à la partie supérieure du corps d'un patient, et le dispositif de connexion peut être relié au poignet et comprend des moyens pour convertir le fléchissement du coude en une rotation du poignet afin d'assurer ainsi une supination et une pronation du poignet.

9. Appareil suivant la revendication 8, caractérisé en ce que le dispositif de connexion pouvant être relié au poignet comprend une console (15) sur laquelle est monté à rotation un pignon (21), et une plaque mobile (25) prévue sur le support et portant un pignon conique (19) engrenant avec le pignon (21), de sorte que le fléchissement du bras à l'endroit du coude provoque une rotation du poignet autour de son axe.

10. Appareil suivant la revendication 1, caractérisé en ce que le dispositif de connexion comprend une manchette reliant une structure de support ($A_4$) à une main, et en ce que le dispositif de fléchissement comprend un fil (425) se déplaçant à travers un guide, le dispositif moteur susdit comprenant un moteur à engrenage (447) et des moyens (432, 433, 435) entraînés par le moteur (447) pour impartir un mouvement de va-et-vient au fil (425), une extrémité de ce fil (425) étant relié à au moins un doigt d'une main.

11. Appareil suivant la revendication 10, caractérisé en ce que le moteur entraîne une chaîne sans fin (433) et le fil (425) est relié à cette chaîne (433) pour se déplacer avec elle afin de subir un mouvement de va-et-vient.

12. Appareil suivant l'une quelconque des revendications 1 à 9, caractérisé en ce que le dispositif moteur (31) est essentiellement en alignement avec le déplacement linéaire susdit et est supporté de manière fixe par le support en vue d'assurer la commande du déplacement de l'élément mobile de manière que l'appareil soit transportable.

FIG.1.

FIG. 2.

C

17

19

15

21

A

23

25

0 078 093

FIG.3.

57 48 43 33 31 51
56 37 46 32 35 39 45 41 A 49 47 53

3

0 078 093

0 078 093

FIG.4.

FIG.5.

FIG. 6.

FIG. 7.

0 078 093

FIG.8.